# FASCICULE DE BREVET EUROPEEN

(11) **EP 2 866 750 B1**
(45) Date de publication et mention de la délivrance du brevet: **11.05.2016**
(21) Numéro de dépôt: 13731333.4
(22) Date de dépôt: 25.06.2013
(51) Int. Cl.: A61F 9/02, G02B 27/01

(54) **DISPOSITIF D'OBSERVATION ET DE PROTECTION CONTRE LES RAYONNEMENTS OPTIQUES ET LES PROJECTIONS SOLIDES OU LIQUIDES**
VORRICHTUNG ZUM BETRACHTEN UND ZUM SCHUTZ GEGEN OPTISCHE STRAHLUNG SOWIE FESTER ODER FLÜSSIGER SPRAY
DEVICE FOR VIEWING AND FOR PROTECTION AGAINST OPTICAL RADIATION AND SOLID OR LIQUID SPRAY

(30) Priorité: 27.06.2012 FR 1256115
(43) Date de publication de la demande: 06.05.2015
(73) Titulaire: Commissariat à l'Énergie Atomique et aux Énergies Alternatives, 75015 Paris (FR)
(72) Inventeur: PIOMBINI, Hervé, 37320 Esvres Sur Indre (FR)
(74) Mandataire: Brevalex
(86) Numéro de dépôt international: PCT/EP2013/063201
(87) Numéro de publication internationale: WO 2014/001287

(56) Documents cités:
- EP-A2- 1 148 718
- FR-A1- 2 954 090
- FR-A1- 2 954 091
- US-A- 5 825 539
- US-A1- 2008 088 529

## Description

### DOMAINE TECHNIQUE

La présente invention concerne un dispositif d'observation et de protection contre les rayonnements optiques, en particulier les rayonnements optiques artificiels qui dépassent des limites d'exposition permises, définies par des normes, et contre les projections solides ou liquides.

Ce dispositif peut consister en des lunettes de protection des yeux contre les rayonnements optiques et les projections solides ou liquides ; et ces lunettes peuvent être aussi bien des lunettes de sécurité à branches que des lunettes-masque.

Mais le dispositif peut aussi consister en un masque couvrant le visage pour protéger cette partie du corps contre les rayonnements optiques et les projections solides ou liquides.

Par « rayonnements optiques », on entend les rayonnements ultraviolets, les rayonnements visibles et les rayonnements infrarouges.

La présente invention s'applique notamment à la protection universelle des travailleurs vis-à-vis des sources de forte luminance qui peuvent être cohérentes (lasers) ou incohérentes (lampes à arc ou arcs de soudure) et vis-à-vis des nouvelles sources lumineuses telles que les sources lasers multispectrales, les diodes électroluminescentes, ou LED, de type blanc froid (en anglais, *cold white light electroluminescent diodes*)*,* les insolateurs, par exemple ceux qui utilisent un rayonnement ultraviolet, et les lasers blancs qui ont de larges spectres.

Ces spectres rendent inefficaces de nombreuses lunettes de protection vis-à-vis des lasers, lunettes qui sont fondées sur des filtres colorés ou des filtres multicouches.

L'invention s'applique également à la protection contre toute intrusion, dans les yeux, d'objets, par exemple des copeaux métalliques ou des particules projetées, ou de projections de liquides.

L'invention peut également être adaptée à l'aide aux personnes malvoyantes qui nécessitent une augmentation de la luminosité ambiante pour voir ou distinguer des objets ainsi que l'environnement de ces objets.

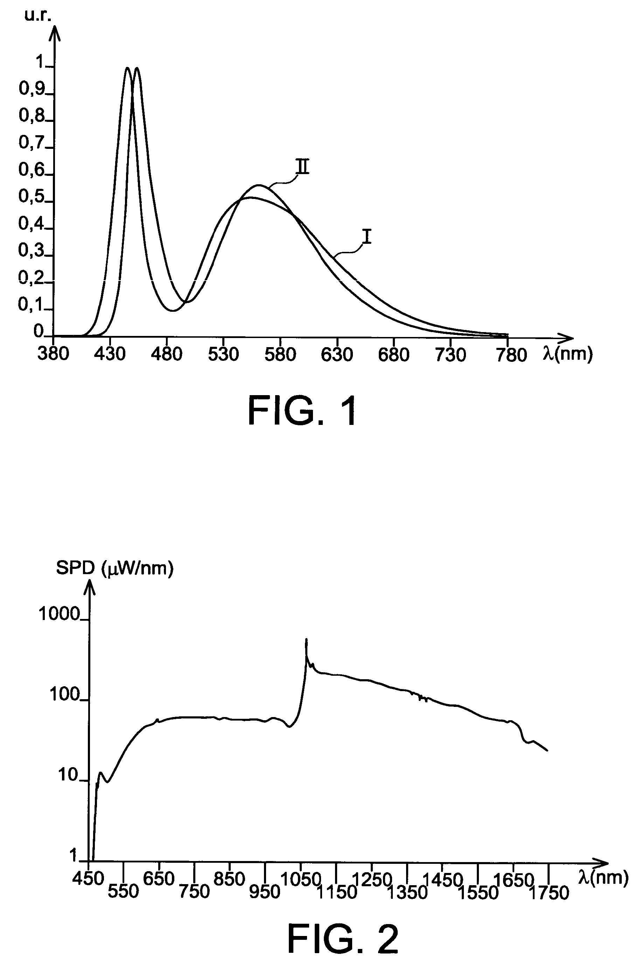

A titre d'exemple des sources mentionnées plus haut, la figure 1 montre deux spectres I et II de systèmes d'éclairage à LED de type blanc froid (axe des abscisses : longueurs d'onde λ en nm ; axe des ordonnées : unités relatives u.r.). Et la figure 2 montre le spectre du rayonnement émis par un laser blanc (axe des abscisses : longueurs d'ondes en nm ; axe des ordonnées : densité de puissance spectrale (en anglais, *spectral power density*) SPD en µW/nm).

Une directive du parlement européen demande d'ailleurs l'introduction de mesures de protection pour les travailleurs contre les rayonnements optiques artificiels qui, à ce jour, n'existent pas en tant que protection individuelle pour les yeux. A ce sujet, on se reportera au document suivant :
**Directive 2006/25/EC of the European Parliament and of the Council of 5 April 2006 on the minimum health and safety requirements regarding the exposure of workers to risks arising from physical agents (artificial optical radiation).**

La présente invention rentre notamment dans le cadre de la protection oculaire individuelle et universelle pour toutes les sources de rayonnements optiques cohérents ou incohérents qui dépassent les limites d'exposition permises, indiquées dans la réglementation européenne EN 60825-1 qui est relative à la sécurité des appareils à laser, ou dans les réglementations internationales telles que celles qui sont données par l'ICNIRP (International Commission on Non-Ionizing Radiation Protection).

### ÉTAT DE LA TECHNIQUE ANTÉRIEURE

Les techniques actuelles de la protection oculaire individuelle contre les lasers sont fondées sur le port de lunettes équipées de filtres colorés.

Un filtre de ce type fonctionne par absorption dans une bande spectrale spécifique, liée à la longueur d'onde du laser, tout en permettant la vision de la scène observée, grâce à la zone de transparence spectrale du filtre utilisé.

De plus, il peut arriver que les filtres employés ne remplissent plus leur fonction de protection dans le cas de flux lumineux importants, à cause de phénomènes d'absorption saturable. A ce sujet, on se reportera par exemple au document suivant :
**Laser damage tests and optical densities of laser goggles,** J. Hue, C. Pelle, P. Garrec, O. Lartigue, F. Baume et J.L. Rochas, Laser-induced damage in optical materials : 1999, Boulder, Colorado, USA, 4-7 Oct. 1999, Proc. of the SPIE, vol. 3902, pp. 500-511**.**

On peut aussi utiliser des filtres multicouches fonctionnant en réflexion mais leur réponse spectrale varie en fonction de l'angle d'incidence et donc en fonction de l'orientation des lunettes lors du mouvement de la tête de l'utilisateur.

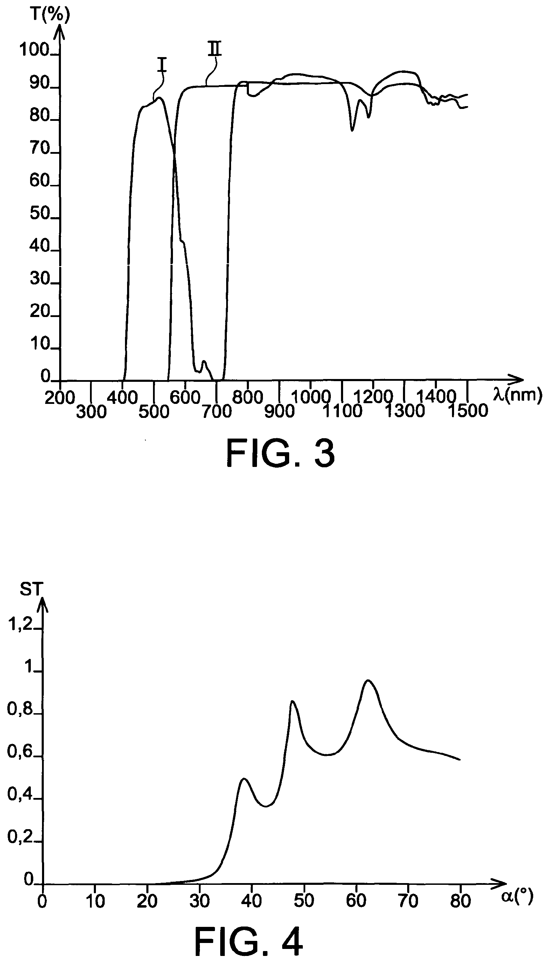

A titre d'exemple, la figure 3 montre deux spectres en transmission de lunettes de protection laser connues, pour un laser HeNe (I) et pour un laser excimère (II) (axe des abscisses : longueurs d'ondes λ en nm ; axe des ordonnées : transmission T en %). Et la figure 4 montre la transmission spectrale ST d'un filtre centré sur 1064 nm, en fonction de l'angle d'incidence α exprimé en degrés.

Par ailleurs, le choix des lunettes de protection qu'il faut utiliser avec une source de rayonnement est effectué (1) à partir de données provenant de cette source, données que l'on ne possède pas nécessairement, et (2) selon des règles issues de normes européennes qui sont relatives à la protection individuelle de l'oeil contre les rayonnements laser mais ne traitent pas le cas des sources multispectrales ni le cas des spectres continus.

Une source multispectrale peut être constituée de plusieurs lasers ayant différentes longueurs d'ondes ; il s'agit par exemple de la juxtaposition d'un laser excimère (fonctionnant dans le domaine ultraviolet), d'un laser à argon (fonctionnant dans le domaine visible) et d'un laser YAG (fonctionnant dans le domaine infrarouge) dans la même zone de travail.

Et les spectres continus sont par exemple les spectres des lasers blancs ou ceux des LED, en particulier des LED qui sont utilisées pour l'éclairage, ou encore ceux des émissions des plasmas tels que les arcs électriques par exemple, qui ont un spectre continu et dont les raies d'émission sont caractéristiques des matériaux en présence.

En outre, les calculs à entreprendre pour choisir les lunettes de protection sont souvent longs et fastidieux pour un non spécialiste, lorsqu'on se ramène aux valeurs d'exposition permises pour l'oeil (voir la norme EN 207 ou EN 208).

De plus, avant d'utiliser des équipements de protection, il faut toujours vérifier que ceux-ci sont appropriés aux rayonnements rencontrés : on risque par exemple de se tromper de lunettes dans le cas de lasers multiples, ou d'utiliser des lunettes détériorées. En effet, ces dernières peuvent par exemple comporter une couche délaminée, un filtre fendu, des rayures ou des impacts.

Les documents FR 2 954 090 et FR 2 954 091 décrivent des lunettes de protection oculaire, comprenant des écrans de restitution d'image, disposés directement devant les yeux d'un utilisateur.

Ces écrans sont placés trop près des yeux pour permettre une vision correcte de la scène observée.

### EXPOSÉ DE L'INVENTION

La présente invention a pour but de remédier aux inconvénients précédents.

Pour ce faire, on prévoit un écran opaque dans un masque ou deux écrans opaques dans des lunettes de protection et l'on associe, à cet écran ou à ces écrans, un dispositif permettant d'acquérir l'image d'une scène et de restituer l'image acquise à l'utilisateur du masque ou des lunettes.

L'invention utilise avantageusement des composants optiques miniaturisés qui sont à présent classiques du fait du développement des téléphones portables auxquels est intégré un appareil photo numérique.

De plus, dans l'invention, on utilise avantageusement des techniques du genre de celles qui permettent la réalisation de lunettes destinées aux jeux vidéo, à la télévision ou au cinéma en trois dimensions, et comportant des dispositifs électroniques commandables, de poids très faible, qui sont insérés dans les branches de ces lunettes.

De façon précise, la présente invention a pour objet un dispositif d'observation et de protection, pour protéger au moins les yeux contre les rayonnements optiques et les projections solides ou liquides, comprenant :
- au moins un écran qui est opaque aux rayonnements optiques et résistant aux projections solides ou liquides, et qui est adapté pour empêcher les rayonnements optiques et les projections solides ou liquides d'atteindre au moins les deux yeux d'un utilisateur, et
- un dispositif d'acquisition et de restitution pour acquérir une image d'une scène et restituer l'image acquise à l'utilisateur.

Lorsque ce dispositif d'acquisition et de restitution est alimenté par une batterie, on prévoit de préférence un dispositif d'alerte pour prévenir l'utilisateur de l'état de charge de cette batterie.

Le dispositif d'acquisition et de restitution comprend :
- au moins une optique de formation d'image, qui est de préférence pourvue d'un système autofocus et qui est rendue rigidement solidaire de l'écran et associée à un dispositif d'imagerie, pour acquérir l'image de la scène, et
- au moins un dispositif de restitution pour restituer l'image acquise à l'utilisateur.

Le dispositif, objet de l'invention, est caractérisé en ce que le dispositif de restitution comprend un dispositif de projection d'image de la scène dans l'oeil de l'utilisateur.

L'ouverture de l'optique de formation d'image peut être commandée automatiquement pour gérer le flux optique ainsi que la position grâce à un système autofocus permettant à l'utilisateur une vision de près.

Selon un premier mode de réalisation particulier de l'invention :
- le dispositif d'acquisition et de restitution comprend une seule optique de formation d'image, qui est rendue rigidement solidaire de l'écran, et un seul dispositif de restitution qui est associé à l'un des deux yeux de l'utilisateur, et
- la distance focale de l'optique de formation d'image est sensiblement égale à la distance focale de l'oeil auquel est associé le dispositif de restitution.

De préférence, l'optique de formation d'image est associée à un capteur d'image de type CCD qui peut avoir un contrôle automatique de gain et une gestion de son diaphragme d'ouverture pour adapter le niveau d'éclairement de la zone observée au juste besoin pour une observation correcte par l'utilisateur.

Selon un deuxième mode de réalisation particulier de l'invention :
- le dispositif d'acquisition et de restitution comprend deux optiques de formation d'image, qui sont rendues rigidement solidaires de l'écran et aptes à permettre une vision stéréoscopique de la scène, et deux dispositifs de restitution qui sont respectivement associés aux deux yeux de l'utilisateur et coopèrent avec les deux optiques de formation d'image et les deux dispositifs d'imagerie associés, pour restituer une image en trois dimensions à l'utilisateur, et
- le quotient de la distance focale de chaque optique de formation d'image par la distance entre les deux optiques de formation d'image est sensiblement égal au quotient de la distance focale de l'oeil correspondant par la distance interpupillaire.

Le dispositif d'imagerie peut comprendre un faisceau de fibres optiques (en anglais, *optical fiber bundle*) (destiné à l'imagerie).

Un tel faisceau est intéressant dans des cas particuliers où le flux optique gênant peut être fortement atténué par des systèmes à fibres optiques (fibres en verre pour des émissions dans l'ultraviolet profond, fibres en silice pour des rayonnements infrarouges à 10,6 µm), ou pour protéger l'utilisateur vis-à-vis de projections solides ou liquides ou même pour aider un utilisateur malvoyant.

En variante, le dispositif d'imagerie comprend une caméra numérique qui permet au système d'augmenter ou de diminuer le contraste de l'image et donc de gérer le gain de l'image (CAG : contrôle automatique de gain) et qui peut être munie, ou non, d'un filtre optique adapté à la longueur d'onde ou à une plage de longueurs d'onde pour atténuer ou éliminer le flux optique provenant de ce domaine spectral. Dans ce cas, le dispositif de restitution comprend de préférence un écran de visualisation (en anglais, *display device*).

De préférence, le dispositif de restitution comprend en outre un dispositif de correction de la vision de l'utilisateur, afin d'obtenir une image nette de la scène dans l'oeil.

Ce dispositif de correction peut être choisi parmi les dispositifs optiques variables et les MOEMS c'est-à-dire les systèmes micro-opto-électromécaniques (en anglais, *micro-opto-electro-mechanical systems*).

Le dispositif, objet de l'invention, peut comprendre en outre un amplificateur de brillance (en anglais, *image intensifier*) pour augmenter la luminosité de l'image, notamment pour aider les malvoyants ou des personnes ayant par exemple des problèmes de cataracte.

Un système de focalisation automatique du genre de ceux que l'on trouve dans les appareils photographiques pourrait être ajouté (triangularisation) afin que l'utilisateur possède toujours une image nette de la zone qu'il observe.

En outre, on peut prévoir un système d'alerte, soit par affichage à l'aide d'une diode verte ou rouge par exemple soit par un buzzer donnant à l'utilisateur l'état de charge d'une batterie qui peut être utilisée dans le dispositif afin que cet utilisateur ne se retrouve pas subitement sans vision de son environnement.

### BRÈVE DESCRIPTION DES DESSINS

La présente invention sera mieux comprise à la lecture de la description d'exemples de réalisation donnés ci-après, à titre purement indicatif et nullement limitatif, en faisant référence aux dessins annexés sur lesquels :
- la figure 1 montre deux spectres de systèmes d'éclairage à LED de type blanc froid et a déjà été décrite,
- la figure 2 montre le spectre du rayonnement émis par un laser blanc et a déjà été décrite,
- la figure 3 montre deux spectres en transmission de lunettes de protection laser connues et a déjà été décrite,
- la figure 4 montre la transmission spectrale d'un filtre centré sur 1064 nm en fonction de l'angle d'incidence et a déjà été décrite,
- la figure 5 est une vue de face schématique et partielle d'un premier mode de réalisation particulier de l'invention,
- la figure 6 est une vue de dessus schématique et partielle de ce premier mode de réalisation particulier,
- les figures 7 et 8 illustrent de façon schématique et partielle la compensation de certains défauts visuels dans une variante de ce premier mode de réalisation particulier,
- la figure 9 est une vue schématique et partielle d'une autre variante de ce premier mode de réalisation particulier,
- la figure 10 est une vue de face schématique et partielle d'un deuxième mode de réalisation particulier de l'invention,
- la figure 11 est une vue de dessus schématique et partielle de ce deuxième mode de réalisation particulier,
- la figure 12 illustre de façon schématique et partielle une variante du premier mode de réalisation particulier,
- la figure 13 illustre de façon schématique et partielle une variante du deuxième mode de réalisation particulier, et
- la figure 14 illustre de façon schématique et partielle un autre exemple de l'invention.

### EXPOSÉ DÉTAILLÉ DE MODES DE RÉALISATION PARTICULIERS

On décrit ci-après divers exemples du dispositif, objet de l'invention. Dans ces exemples, le dispositif consiste en des lunettes de protection des yeux contre les rayonnements optiques et les projections solides ou liquides.

La restitution d'une scène à un utilisateur de ces lunettes de protection est effectuée par un dispositif du genre des dispositifs d'affichage tête haute (en anglais, *head up displays*).

Une image peut ainsi être projetée pour l'un des yeux de l'utilisateur (ou pour chaque oeil de celui-ci), à l'aide d'un mini-miroir centré sur l'axe optique de l'oeil (ou de deux mini-miroirs respectivement centrés sur les axes optiques des yeux).

On peut aussi utiliser des dispositifs plus intégrés, comportant un ensemble de réseaux prismatiques du genre de ceux qui sont développés par la Société Optinvent.

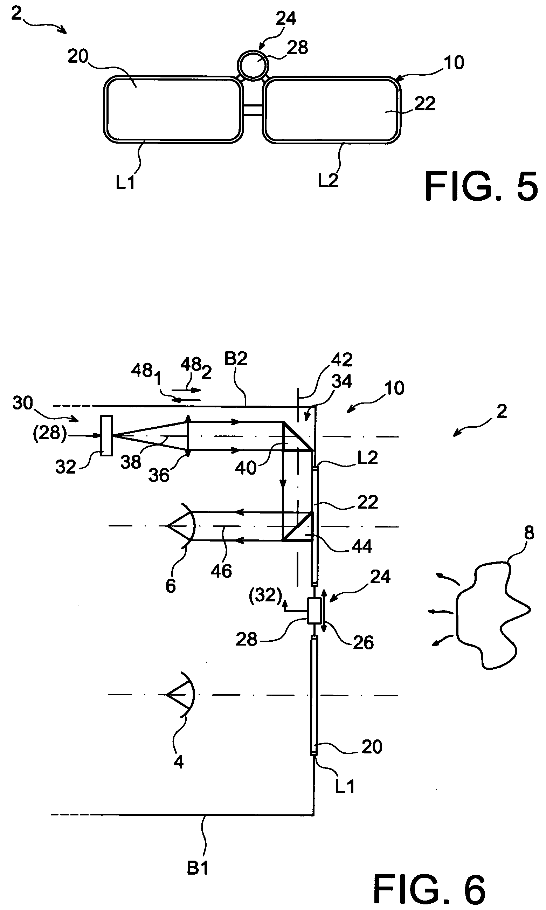

Un premier mode de réalisation particulier de l'invention est illustré par les figures 5 et 6.

La figure 5 est une vue de face schématique et partielle de ce premier mode de réalisation particulier; et la figure 6 en est une vue de dessus schématique et partielle.

Ces figures 5 et 6 montrent des lunettes 2 destinées à protéger les yeux 4 et 6 d'un utilisateur de ces lunettes, en train d'observer une scène 8, contre les rayonnements optiques et les projections solides ou liquides.

Ces lunettes sont pourvues d'un masque (non représenté) qui empêche des rayonnements latéraux ou des projections latérales d'atteindre l'utilisateur.

Les alimentations électriques qui sont nécessaires au fonctionnement des lunettes (par fil ou par batterie) peuvent être comprises dans ce masque.

Les lunettes 2 comprennent une monture 10 et, conformément à l'invention, elles comprennent en outre :
- deux écrans 20 et 22 qui sont opaques aux rayonnements optiques et résistants aux projections solides ou liquides, sont fixés à la monture et sont respectivement positionnés pour empêcher les rayonnements optiques et les projections solides ou liquides d'atteindre les deux yeux 4 et 6 de l'utilisateur, et
- un dispositif d'acquisition et de restitution 24, destiné à acquérir une image de la scène 8 et à restituer l'image acquise à l'utilisateur.

Les deux écrans peuvent être faits d'un polymère (par exemple le PMMA) teinté dans sa masse ou d'un oxyde réfractaire ou d'un métal, en particulier d'un métal du type aluminium oxydé noir dans le cas de lasers de forte puissance qui peuvent induire une fusion du polymère ou une fissuration de l'oxyde réfractaire et donc entraîner un risque pour l'utilisateur.

Dans l'exemple représenté, les lunettes 2 comprennent aussi deux logements d'oculaires (en anglais, *eyepieces*) L1 et L2 qui sont prévus dans la monture 10, cette dernière est pourvue de deux branches qui sont symbolisées par les lignes B1 et B2, et les deux écrans 20 et 22 sont respectivement montés dans les deux logements d'oculaires L1 et L2.

Dans cet exemple, le dispositif 24 comprend :
- une optique de formation d'image 26, fixée à la monture 10, au centre de celle-ci, et faisant partie d'une caméra numérique 28 formant un dispositif d'imagerie (active), pour acquérir l'image de la scène 8 avec un bon niveau de contraste grâce à une adaptation automatique du gain de la caméra ou de l'ouverture de l'optique, et
- un dispositif de restitution 30, destiné à restituer l'image acquise à l'utilisateur.

Ce dispositif 30 est associé à l'un des deux yeux de l'utilisateur. Dans l'exemple représenté, il s'agit de l'oeil gauche 6.

De plus, la distance focale F_{c} de l'optique 26 de la caméra 28 est sensiblement égale à la distance focale Fₒ de l'oeil 6, qui vaut environ 17 mm.

La caméra numérique 28, ou les deux caméras numériques dans les exemples donnés plus loin, sont bien entendu adaptées aux rayonnements optiques considérés.

Avec des rayonnements infrarouges, on peut adjoindre des mini-caméras numériques pour l'infrarouge pour visualiser les impacts de faisceaux sur les matériaux. Avec des rayonnements ultraviolets, on peut adjoindre des mini-caméras numériques pour l'ultraviolet pour visualiser les impacts de faisceaux sur les matériaux. Et avec des rayonnements visibles, on utilise des mini-caméras numériques adaptées à de tels rayonnements; on peut alors utiliser des caméras noir et blanc ou des caméras couleurs.

Bien entendu, le ou les écrans de visualisation restitueront une image visible de la scène observée.

Le dispositif de restitution 30 comprend un écran de visualisation 32 qui est électriquement connecté à la caméra 28 et prévu pour afficher l'image de la scène 8 qui a été formée dans la caméra 28 par l'intermédiaire de l'optique 26.

L'écran de visualisation, ou les deux écrans de visualisation dont il sera question par la suite, peuvent être choisis parmi les écrans à cristaux liquides (en anglais, *liquid crystal display*) ou LCD, les écrans à plasma et les écrans à diodes électroluminescentes organiques (en anglais, *organic light emitting diodes*) ou OLED.

Le dispositif 30 comprend aussi un dispositif 34 de projection d'image de la scène 8 dans l'oeil 6 de l'utilisateur. En effet, l'image de la scène 8 est affichée en direction de cette dernière par l'écran de visualisation 32 et l'oeil 6 regarde aussi en direction de la scène 8.

Comme on le voit sur la figure 6, dans l'exemple décrit, le dispositif de projection 34 comprend :
- une lentille de reprise d'image 36, dont l'axe optique a la référence 38,
- un prisme 40 disposé sur l'axe 38 pour dévier à 90° la lumière issue de la lentille 36, suivant un axe (géométrique) 42,
- un miroir à 45° 44 pour diriger cette lumière vers l'oeil 6 de l'utilisateur, suivant l'axe optique 46 de cet oeil.

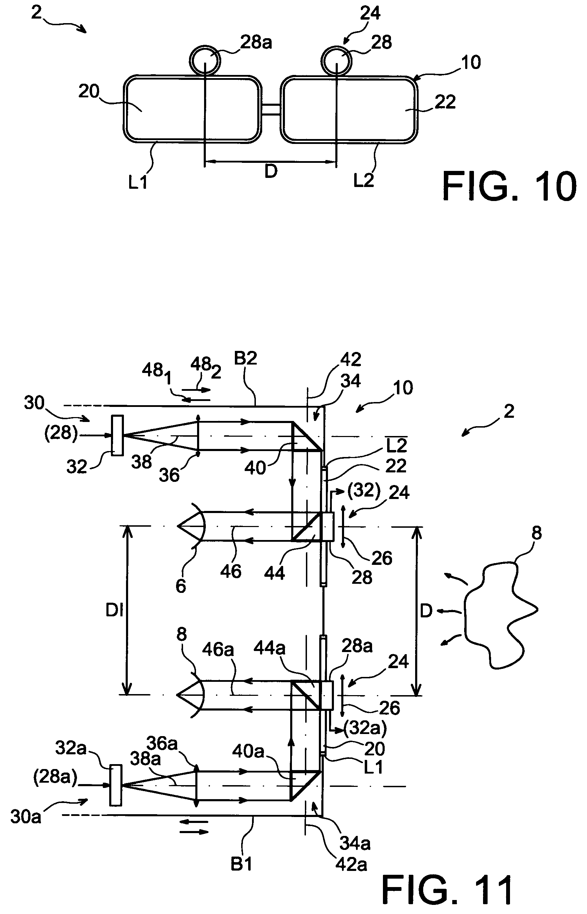

Le miroir 44, ou les miroirs si l'on en utilise plusieurs pour assurer une fonction de réflexion (voir en particulier l'exemple des figures 10 et 11) peuvent être de type diélectrique. Cela permet d'avoir le contraste d'image souhaité selon la valeur du coefficient de réflexion choisi, et en particulier de pouvoir obtenir une forte efficacité, proche de 100%.

Mais on peut également utiliser de simples miroirs métalliques compte tenu du faible coût de ceux-ci.

De préférence, le dispositif de restitution 30 comprend en outre un dispositif prévu pour corriger la vision de l'utilisateur, en cas de myopie, d'hypermétropie ou d'astigmatisme de celui-ci, afin d'obtenir une image nette de la scène dans l'oeil 6.

La lentille 36 est alors une lentille appropriée, simple ou multiple. On veut par exemple utiliser un système optique de révolution qui peut être chromatique ou non, selon la résolution souhaitée poru l'image visualisée.

On peut aussi introduire une lentille cylindrique de puissance donnée, dont les axes de correction sont orientés suivants l'oeil considéré de l'utilisateur en vue de corriger son astigmatisme le cas échéant. Bien entendu, dans les exemples de l'invention où chaque oeil reçoit une image, comme dans l'exemple des figures 10 et 11, on utilise deux telles lentilles cylindriques et chacune d'elles a ses axes de corrections qui sont orientés suivant l'oeil qui lui est associé.

Dans l'exemple, le dispositif de correction est un dispositif optique variable : la lentille 36 est pourvue de moyens pour la déplacer suivant son axe optique 38, moyens symbolisés par les flèches 48₁ (cas d'un utilisateur myope) et 48₂ (cas d'un utilisateur hypermétrope) sur la figure 6.

Le prisme 40 permet une meilleure stabilité de l'image lors des mouvements de l'observateur (utilisateur) et une conservation de la direction du faisceau plus ou moins indépendant de la position des branches des lunettes.

On peut aussi prévoir des moyens de rotation de la lentille 36 autour de son axe optique 38 pour effectuer les corrections. Si les lunettes de protection sont destinées à être utilisées par plusieurs personnes, on prévoit alors une rotation variable de la lentille 36 pour que chaque personne puisse choisir les corrections d'astigmatisme qui lui conviennent, et une translation de cette lentille 36 pour corriger les différents défauts de mise au point, liés aux différentes personnes (observateurs de scènes).

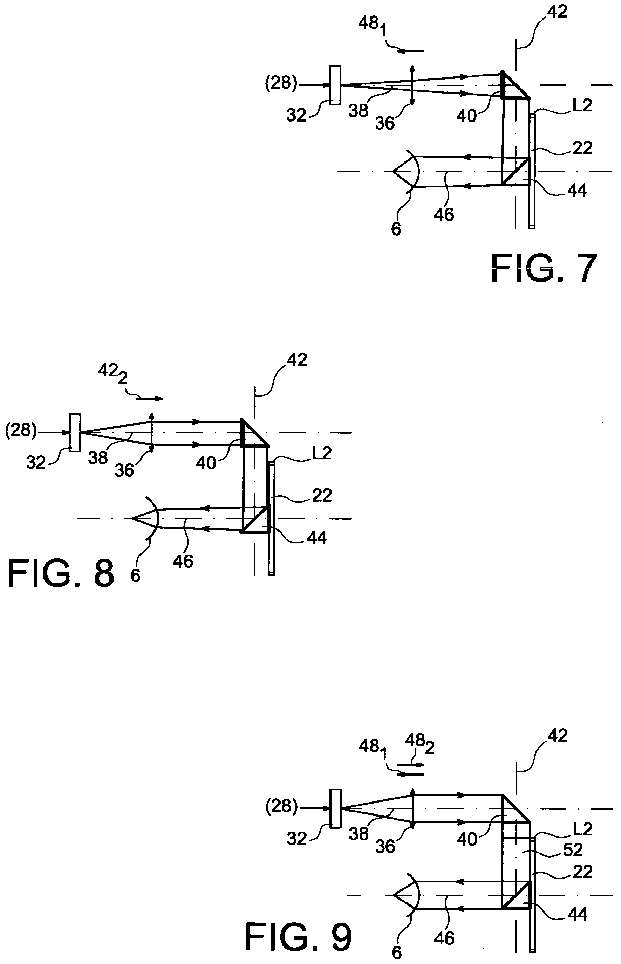

Les figures 7 et 8 illustrent de façon schématique et partielle le déplacement de la lentille 36 pour compenser certains défauts visuels de l'utilisateur. Sur ces figures, on a seulement repris certains éléments de la figure 6 (avec les mêmes références).

On précise que le déplacement de la lentille peut être fixe, dans le cas où les lunettes de protection sont destinées à un seul utilisateur, ou au contraire réglable dans le cas où ces lunettes peuvent être utilisées par plusieurs personnes.

La figure 7 illustre une correction dans le cas où l'oeil 6 est myope. Alors, la lentille 36 est approchée de l'écran de visualisation 32 suivant la flèche 48₁.

La figure 8 illustre, quant à elle, une correction dans le cas où l'oeil 6 est hypermétrope. Alors, la lentille 36 est éloignée de l'écran de visualisation 32 suivant la flèche 48₂.

Les corrections visuelles de l'utilisateur (ou des utilisateurs), mentionnées précédemment, peuvent aussi être effectuées au moyen de MOEMS prévus à cet effet.

La figure 9 illustre de façon schématique et partielle la possibilité de remplacer le miroir 44 de la figure 6 par un prisme 52 qui travaille en réflexion totale et qui est prévu pour recevoir la lumière du prisme 40 suivant l'axe 42 et pour dévier cette lumière vers l'oeil 6 suivant l'axe optique 46. Sur cette figure, on a seulement repris certains éléments de la figure 6 (avec les mêmes références).

On décrit ci-après un deuxième mode de réalisation particulier de l'invention en faisant référence aux figures 10 et 11.

La figure 10 est une vue de face schématique et partielle de ce deuxième mode de réalisation particulier; et la figure 11 en est une vue de dessus schématique et partielle.

Comme on le voit, dans ce deuxième mode de réalisation particulier, les lunettes de protection 2 comprennent encore la monture 10, pourvue des logements d'oculaires 12 et 14 et des branches 16 et 18 ; et les écrans 20 et 22 sont encore montés dans les logements d'oculaires 12 et 14, pour protéger les yeux 4 et 6 de l'utilisateur, comme on l'a expliqué dans la description des figures 5 et 6.

La différence entre les deux modes de réalisation particuliers réside dans le dispositif d'acquisition et de restitution 24, destiné à acquérir une image de la scène 8 observée par l'utilisateur des lunettes, et à restituer à ce dernier l'image acquise : dans l'exemple des figures 10 et 11, on utilise deux caméras numériques 28 et 28a au lieu de l'unique caméra numérique 28 des figures 5 et 6, en vue de restituer une image en trois dimensions de la scène 8 à l'utilisateur.

La caméra numérique 28 correspond à l'oeil gauche 6 de l'utilisateur et la caméra numérique 28a à l'oeil droit 4 de celui-ci. De plus, la caméra numérique 28 est associée au dispositif de restitution 30 qui a été précédemment décrit en faisant référence à la figure 6 et utilise donc les mêmes composants avec les mêmes références comme on le voit.

La caméra numérique 28a est, quant à elle, associée à un autre dispositif de restitution 30a. Ce dernier est constitué comme le dispositif 30 mais, pour distinguer les composants respectifs de ces dispositifs 30 et 30a, ceux du dispositif 30a portent les références des composants du dispositif 30, suivies de la lettre a comme on le voit sur la figure 11.

Les deux dispositifs d'imagerie formés par les deux caméras numériques 28 et 28a sont respectivement pourvus de deux optiques de formation d'image identiques 26 et 26a.

Ces objectifs 26 et 26a peuvent être commandés pour gérer leur ouverture, permettant d'acquérir plus ou moins de flux, ou gérer leur mise au point par un système d'autofocus. Ils peuvent, le cas échéant, être munis de filtres optiques pour protéger le capteur d'image.

Ces objectifs (et les caméras numériques associées) sont fixés à la monture 10, de part et d'autre du centre de celle-ci et de manière à permettre une vision stéréoscopique de la scène 8.

Les deux dispositifs de restitution 30 et 30a qui sont respectivement associés aux deux yeux 6 et 4 de l'utilisateur, coopèrent ainsi avec les deux optiques 26 et 26a et les deux caméras numériques associées 28 et 28a, pour restituer une image en trois dimensions à l'utilisateur.

Le miroir 44 ou le prisme 52 sont centrés sur l'axe de vision (ou axe optique) de l'utilisateur par déplacement mécanique (de type binoculaire de microscope) ou par un centrage par rapport à la monture, comme un verre de lunette classique, pour respecter l'écart pupillaire de l'utilisateur.

On précise en outre que les caméras numériques sont respectivement centrées sur les axes optiques des yeux de l'utilisateur si la focale de l'objection a la même focale que celle de l'oeil humain à savoir 17 mm. La position relative des caméras situées sur les lunettes, relative dépend donc de la distance interpupillaire DI, ou écart interpupillaire, de l'utilisateur et de la focale de l'objectif de la caméra, pour que ce dernier retrouve une parfaite vision du relief. Les caméras suivent donc parfaitement les mouvements de la tête de l'utilisateur.

On précise aussi que la condition suivante est respectée afin de conserver une bonne impression du relief: le quotient de la distance focale F_{c} (respectivement F_{c}a) de l'optique de formation d'image 26 (respectivement 26a) par la distance D (figures 10 et 11) entre les deux optiques 26 et 26a est sensiblement égal au quotient de la distance focale Fₒ (respectivement Fₒa) de l'oeil correspondant 6 (respectivement 4) par la distance interpupillaire DI qui va d'environ 57 mm à 65 mm (figure 11).

En fait, dans l'exemple considéré, les optiques 26 et 26a sont identiques et ont donc la même distance focale F_{c}. Ainsi, la condition ci-dessus devient : F_{c}/D sensiblement égal à Fₒ/DI et à Fₒa/DI. Et, en considérant que les yeux ont sensiblement la même distance focale Fₒ (qui vaut environ 17 mm) et en choisissant D sensiblement égal à DI, la condition devient : F_{c} sensiblement égal à Fₒ.

L'ajustement de F_{c}/D à Fₒ/DI peut être effectué, par exemple, en usinant les deux écrans opaques 20 et 22, ou par un déplacement mécanique (du type binoculaire de microscope).

Bien entendu, on prévoit de préférence un dispositif de correction de vision pour chaque oeil de l'utilisateur, du même genre que le dispositif décrit plus haut : on prévoit alors des moyens pour déplacer chacune des deux lentilles 36 et 36a.

Les prismes 40 et 40a permettent une meilleure stabilité de l'image lors des mouvements de l'observateur, et une conservation de la direction du faisceau plus ou moins indépendant de la position des branches des lunettes.

Chacun des miroirs 44 et 44a peut être remplacé par un prisme du genre du prisme 52, comme on l'a expliqué plus haut.

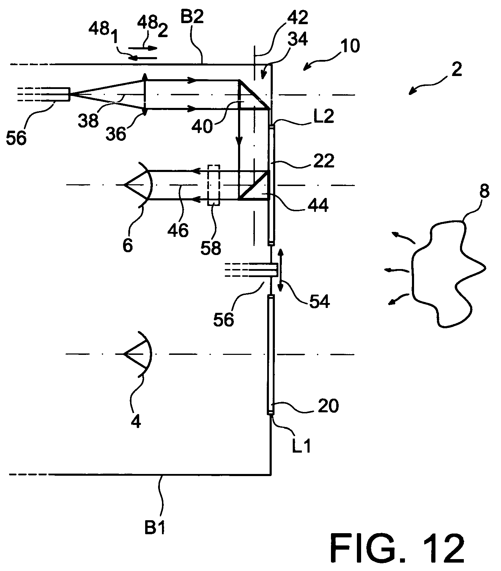

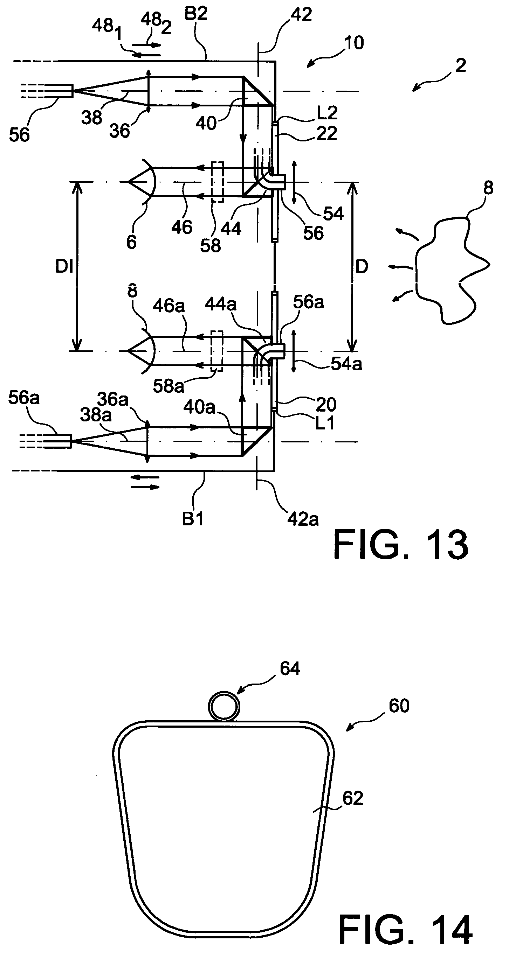

Dans l'exemple des figures 5 et 6, ou dans celui des figures 10 et 11, chaque ensemble comprenant une caméra numérique et un écran de visualisation peut être remplacé par un ensemble comprenant un faisceau de fibres optiques pour imagerie (passive). Ceci est schématiquement illustré par la figure 12 qui correspond à la figure 6, et par la figure 13 qui correspond à la figure 11.

Un tel système passif ne sera utilisé que dans le cas où le faisceau de fibres arrête suffisamment, par transmission intrinsèque du matériau constitutif du faisceau de fibres, les rayonnements qui sont nocifs pour les yeux de l'utilisateur.

Sur la figure 12, on voit une optique de formation d'image 54 correspondant à l'optique 26 de la figure 6 et fixée, comme cette dernière, à la monture 10, au centre de cette monture. Une première extrémité d'un faisceau 56 de fibres optiques (par exemple en verre, en silice ou en plastique) est fixée à la monture 10, en regard de l'optique 54.

Cette optique 54 est prévue pour focaliser la lumière provenant de la scène 8 sur la première extrémité du faisceau 56. La deuxième extrémité de celui-ci est fixée en regard de la lentille de reprise d'image 36.

Sur la figure 13, on voit deux optiques de formation d'image 54 et 54a correspondant respectivement aux optiques 26 et 26a de la figure 11 et fixées, comme ces dernières, à la monture 10, de part et d'autre du centre de celle-ci. Une première extrémité d'un faisceau de fibres optiques 56 ou 56a est fixée à la monture 10, de part et d'autre du centre de celle-ci. Une première extrémité d'un faisceau de fibres optiques 56 ou 56a est fixée à la monture 10, en regard de l'optique 54 ou 54a.

Cette optique 54 ou 54a est prévue pour focaliser la lumière provenant de la scène 8 sur la première extrémité du faisceau 56 ou 56a. La deuxième extrémité de celui-ci est fixée en regard de la lentille de reprise d'image 36 ou 36a.

Bien entendu, dans le cas de la figure 12, la distance focale de l'optique 54, que l'on peut encore noter F_{c}, est sensiblement égale à la distance focale Fₒ de l'oeil 6. De même, dans le cas de la figure 13, les conditions mentionnées plus haut, relatives aux paramètres notés D, DI, Fₒ, Fₒa, F_{c} et F_{c}a, s'appliquent ici (avec D : distance entre les optiques 54 et 54a ; et F_{c} ou F_{c}a : distance focale de l'optique 54 ou 54a).

Si on le juge utile, on peut prévoir un amplificateur de brillance 58 (figure 12) pour augmenter la luminosité de l'image avant que cette image ne parvienne à l'oeil 6 de l'utilisateur.

Cet amplificateur de brillance sera en particulier réservé à la protection des yeux vis-à-vis de la projection de matière ou de liquide. Il pourra servir également pour l'aide à la vision pour des gens malvoyants.

De même, on peut prévoir deux amplificateurs de brillance 58 et 58a (figure 13) pour augmenter la luminosité des images avant qu'elles ne parviennent respectivement aux yeux 6 et 4 de l'utilisateur.

De tels amplificateurs de brillance peuvent même être utilisés dans les exemples de l'invention qui emploient des caméras numériques.

On précise que dans les exemples donnés plus haut, le niveau lumineux peut être adapté en agissant sur le gain de la caméra ou des caméras numériques, ou sur l'intensité de l'écran ou des écrans de visualisation, ou sur l'intensité de l'amplificateur ou des amplificateurs de brillance.

Comme on l'a vu, dans les exemples de l'invention, donnés plus haut, la ou les caméras numériques sont fixées à la monture 10 des lunettes de sécurité. Mais tous les autres composants qui équipent celles-ci et permettent d'obtenir un dispositif de vision intégrée (en particulier : faisceau(x) de fibres optiques, prisme(s), miroir(s), lentille(s), écran(s) de visualisation, optique(s)), y compris les dispositifs électroniques et les dispositifs d'alimentation électrique, tels que des batteries, qui peuvent être nécessaires au fonctionnement des lunettes de protection, peuvent être insérées dans les branches ou dans les écrans opaques de celles-ci, ou être intégrés à ces branches ou à ces écrans opaques ou, plus généralement, à la monture.

Si on le juge utile, on pourra intégrer un système d'autofocus afin de permettre l'aide à la vision de près d'utilisateurs malvoyants, ayant une cataracte par exemple.

Dans le cas où l'on utilise un prisme à réflexion totale en association avec l'un des deux yeux, ou deux prismes à réflexion totale, respectivement associés aux deux yeux, chaque prisme peut être placé dans l'axe de rotation ou de fixation de la branche correspondante des lunettes de protection de façon à obtenir une image peu sensible au défaut induit par la mise en place des lunettes.

On dispose ainsi de lunettes de protection pourvues d'un dispositif de vision intégré.

Il convient de noter que certaines lunettes de protection ne comportent pas de branches mais sont pourvues de protections latérales. Dans ce cas, l'insertion ou l'intégration peut être faite dans ces protections latérales ou à ces dernières.

Les exemples de l'invention, que l'on a donnés plus haut, sont relatifs à des lunettes de protection qui permettent de protéger les deux yeux d'un utilisateur contre les rayonnements optiques et les projections solides ou liquides, tout en lui permettant d'observer une scène.

Mais l'invention ne se limite pas à de telles lunettes : on peut concevoir un dispositif conforme à l'invention, constitué par un masque qui permet de protéger la totalité du visage de la personne portant ce masque contre les rayonnements optiques et les projections solides ou liquides, tout en lui permettant d'observer une scène.

L'homme du métier peut adapter les exemples donnés plus haut à la réalisation d'un tel masque dont un exemple est vu de face sur la figure 14 où il a la référence 60 : ce masque comporte un unique écran 62 qui protège le visage et qui est opaque aux rayonnements et résistant aux projections ; et cet écran est encore muni d'un dispositif 64 pour acquérir l'image de la scène et restituer l'image acquise à la personne qui porte le masque.

Comme les lunettes décrites plus haut, le masque peut être adapté à l'aide à la vision pour les malvoyants.

## Revendications

1. Dispositif d'observation et de protection, pour protéger au moins les yeux contre les rayonnements optiques et les projections solides ou liquides, comprenant :
- au moins un écran (20, 22; 62) qui est opaque aux rayonnements optiques et résistant aux projections solides ou liquides, et qui est adapté pour empêcher les rayonnements optiques et les projections solides ou liquides d'atteindre au moins les deux yeux (4, 6) d'un utilisateur, et
- un dispositif d'acquisition et de restitution (24 ; 64) pour acquérir une image d'une scène (8) et restituer l'image acquise à l'utilisateur,
dans lequel le dispositif d'acquisition et de restitution (24) comprend :
- au moins une optique de formation d'image (26, 26a), rendue rigidement solidaire de l'écran (20, 22) et associée à un dispositif d'imagerie (28, 28a ; 56, 56a) pour acquérir l'image de la scène (8), et
- au moins un dispositif de restitution (30, 30a) pour restituer l'image acquise à l'utilisateur,
**caractérisé en ce que** le dispositif de restitution comprend un dispositif (34, 34a) de projection d'image de la scène dans l'oeil de l'utilisateur.

2. Dispositif selon la revendication 1, dans lequel :
- le dispositif d'acquisition et de restitution (24) comprend une seule optique de formation d'image (26), qui est rendue rigidement solidaire de l'écran (20, 22) et un seul dispositif de restitution (30) qui est associé à l'un (6) des deux yeux de l'utilisateur, et
- la distance focale de l'optique de formation d'image (26) est égale à la distance focale, environ 17 mm, de l'oeil (6) auquel est associé le dispositif de restitution (30).

3. Dispositif selon la revendication 1, dans lequel :
- le dispositif d'acquisition et de restitution (24) comprend deux optiques de formation d'image (26, 26a), qui sont rendues rigidement solidaires de l'écran (20, 22) et aptes à permettre une vision stéréoscopique de la scène (8), et deux dispositifs de restitution (30, 30a) qui sont respectivement associés aux deux yeux (6, 4) de l'utilisateur et coopèrent avec les deux optiques de formation d'image et les deux dispositifs d'imagerie associés, pour restituer une image en trois dimensions à l'utilisateur, et
- le quotient de la distance focale de chaque optique de formation d'image (26, 26a) par la distance (D) entre les deux optiques de formation d'image est égal au quotient de la distance focale, environ 17 mm, de l'oeil (6, 4) correspondant, par la distance interpupillaire (DI) qui va d'environ 57 mm à 65 mm.

4. Dispositif selon l'une quelconque des revendications 1 à 3, dans lequel le dispositif d'imagerie comprend un faisceau de fibres optiques (56, 56a).

5. Dispositif selon l'une quelconque des revendications 1 à 3, dans lequel le dispositif d'imagerie comprend une caméra numérique (28, 28a).

6. Dispositif selon la revendication 5, dans lequel le dispositif de restitution comprend un écran de visualisation (32, 32a).

7. Dispositif selon l'une des revendications 1 à 6, dans lequel le dispositif de restitution comprend en outre un dispositif (48₁, 48₂, 50₁, 50₂) de correction de la vision de l'utilisateur, afin d'obtenir une image nette de la scène dans l'oeil.

8. Dispositif selon la revendication 7, dans lequel le dispositif de correction est choisi parmi les dispositifs optiques variables et les systèmes micro-opto-électromécaniques.

9. Dispositif selon l'une quelconque des revendications 1 à 8, comprenant en outre un amplificateur de brillance (58, 58a) pour augmenter la luminosité de l'image.

## Patentansprüche

1. Vorrichtung zur Beobachtung und zum Schützen wenigstens der Augen gegen optische Strahlungen und feste oder flüssige Spritzer, umfassend:
- wenigstens einen Schirm (20, 22; 62), der opak ist für optische Strahlungen und widerstandsfähig für feste oder flüssige Spritzer, und der dazu ausgelegt ist, zu verhindern, dass die optischen Strahlungen und die festen oder flüssigen Spritzer wenigstens die zwei Augen (4, 6) eines Benutzers erreichen, und
- eine Erfassungs- und Wiederherstellungsvorrichtung (24; 64) zum Erfassen eines Bilds einer Szene (8) und zum Wiederherstellen des erfassten Bilds für den Benutzer,
wobei die Erfassungs- und Wiederherstellungsvorrichtung (24) umfasst:
- wenigstens eine Bildformungsoptik (26, 26a) die starr mit dem Schirm (20, 22) verbunden und einer Bildgebungsvorrichtung (28, 28a; 56, 56a) zugeordnet ist, um das Bild der Szene (8) zu erfassen, und
- wenigstens eine Wiederherstellungsvorrichtung (30, 30a) zum Wiederherstellen des erfassten Bilds für den Benutzer, **dadurch gekennzeichnet, dass** die Wiederherstellungsvorrichtung eine Vorrichtung (34, 34a) zur Projektion des Bilds der Szene in das Auge des Benutzers umfasst.

2. Vorrichtung nach Anspruch 1, wobei:
- die Erfassungs- und Wiederherstellungsvorrichtung (24) eine einzige Bildformungsoptik (26) umfasst, die starr mit dem Schirm (20, 22) verbunden ist, sowie eine einzige Wiederherstellungsvorrichtung (30), die einem (6) der zwei Augen des Benutzers zugeordnet ist, und
- die Brennweite der Bildformungsoptik (26) gleich der Brennweite, ungefähr 17 mm, des Auges (6) ist, dem die Wiederherstellungsvorrichtung (30) zugeordnet ist.

3. Vorrichtung nach Anspruch 1, wobei:
- die Erfassungs- und Wiederherstellungsvorrichtung (24) zwei Bildformungsoptiken (26, 26a) umfasst, die starr mit dem Schirm (20, 22) verbunden und dazu ausgelegt sind, eine stereoskopische Betrachtung der Szene (8) zu ermöglichen, sowie zwei Wiederherstellungsvorrichtungen (30, 30a), die jeweilig den zwei Augen (6, 4) des Benutzers zugeordnet sind und mit den zwei Bildformungsoptiken und den zwei zugeordneten Bildgebungsvorrichtungen zusammenwirken, um ein dreidimensionales Bild für den Benutzer wiederherzustellen, und
- der Quotient der Brennweite jeder Bildformungsoptik (26, 26a) geteilt durch den Abstand (D) zwischen den zwei Bildformungsoptiken gleich dem Quotienten der Brennweite, ungefähr 17 mm, des entsprechenden Auges (6, 4) geteilt durch den Zwischenpupillenabstand (DI) ist, der zwischen ungefähr 57 mm und 65 mm liegt.

4. Vorrichtung nach einem der Ansprüche 1 bis 3, bei der die Bildgebungsvorrichtung einen Strahl von optischen Fasern (56, 56a) umfasst.

5. Vorrichtung nach einem der Ansprüche 1 bis 3, bei der die Bildgebungsvorrichtung eine Digitalkamera (28, 28a) umfasst.

6. Vorrichtung nach Anspruch 5, bei der die Wiederherstellungsvorrichtung einen Visualisierungsschirm (32, 32a) umfasst.

7. Vorrichtung nach einem der Ansprüche 1 bis 6, bei der die Wiederherstellungsvorrichtung ferner eine Vorrichtung (48₁, 48₂, 50₁, 50₂) zur Korrektur der Sicht des Benutzers umfasst, um ein scharfes Bild der Szene im Auge zu erzielen.

8. Vorrichtung nach Anspruch 7, bei der die Korrekturvorrichtung ausgewählt ist aus den variablen optischen Vorrichtungen und den mikrooptoelektromechanischen Systemen.

9. Vorrichtung nach einem der Ansprüche 1 bis 8, ferner umfassend einen Brillianzverstärker (58, 58a) zur Erhöhung der Helligkeit des Bilds.

## Claims

1. Observation and protection device for protecting at least the eyes against optical radiations and solid or liquid spray, comprising:
- at least one screen (20, 22; 62) that is opaque to optical radiations and withstanding solid or liquid spray and which is suitable for preventing the optical radiations and solid or liquid spray from reaching at least the two eyes (4, 6) of a user, and
- an acquisition and restoration device (24; 64) for acquiring an image of a scene and restoring the acquired image to the user,
wherein the acquisition and restoration device (24) comprises:
- at least one image-formation optical device (26, 26a) that is rigidly connected to the screen (20, 22) and associated with an imaging device (28, 28a; 56, 56a) in order to acquire the image of the scene (8), and
- at least one restoration device (30, 30a) for restoring the acquired image to the user,
**characterised in that** the restoration device comprises a device (34, 34a) for projecting an image of the scene into the eye of the user.

2. Device according to claim 1, wherein:
- the acquisition and restoration device (24) comprises a single image-formation optical device (26) that is rigidly connected to the screen (20, 22), and a single restoration device (30) that is associated with one of the two eyes (6) of the user, and
- the focal distance of the image-formation optical device (26) is equal to the focal distance, about 17 mm, of the eye (6) with which the restoration device (30) is associated.

3. Device according to claim 1, wherein:
- the acquisition and restoration device (24) comprises two image-formation optical devices (26, 26a) that are rigidly connected to the screen (20, 22) and suitable for affording stereoscopic vision of the scene (8), and two restoration devices (30, 30a) that are respectively associated with the two eyes (6, 4) of the user and cooperate with the two image-formation optical devices and the associated two imaging devices, in order to restore an image in three dimensions to the user, and
- the quotient of the focal distance of each image-formation optical device (26, 26a) and the distance (D) between the two image-formation optical devices is equal to the quotient of the focal distance, about 17mm, of the corresponding eye (6, 4) and the interpupil distance (DI) which goes from about 57 mm to 65 mm.

4. Device according to any one of claims 1 to 3, in which the imaging device comprises a bundle of optical fibres (56, 56a).

5. Device according to any one of claims 1 to 3, in which the imaging device comprises a digital camera (28, 28a).

6. Device according to claim 5, in which the restoration device comprises a display device (32, 32a).

7. Device according to one of claims 1 to 6, in which the restoration device also comprises a device (48₁, 48₂, 50₁, 50₂) for correcting the vision of the user in order to obtain a sharp image of the scene in the eye.

8. Device according to claim 7, in which the correction device is chosen from variable optical devices and micro-opto-electromechanical systems.

9. Device according to any one of claims 1 to 8, further comprising a brightness amplifier (58, 58a) for increasing the luminosity of the image.
